# EUROPEAN PATENT APPLICATION

(11) **EP 2 255 825 A2**
(43) Date of publication of application: **01.12.2010**
(21) Application number: 09704175.0
(22) Date of filing: 23.01.2009
(51) Int. Cl.: A61K 38/22

(54) **COMPOSITION FOR PREVENTING OR TREATING BRAIN DISEASES**

(30) Priority: 25.01.2008 KR 20080008207
(71) Applicant: Regeron, Inc., Gangwon-do 200-161 (KR)
(72) Inventor: LEE, Heejae, Chuncheon-si Gangwon-do 200-150 (KR); BYUN, Jong-Seon, Chuncheon-si Gangwon-do 200-770 (KR); LEE, Kyunyoung, Chuncheon-si Gangwon-do 200-160 (KR); BAIK, Sangjung, Chuncheon-si Gangwon-do 200-160 (KR); OH, Dahlkyun, Chuncheon-si Gangwon-do 200-160 (KR)
(74) Representative: Winkler, Andreas Fritz Ernst
(86) International application number: PCT/KR2009/000364
(87) International publication number: WO 2009/093864

(57) **Abstract**

The present invention relates to a composition for preventing or treating neurological diseases, particularly brain diseases and improving cognitive functions by inhibiting apoptosis of neuronal cells and/or promoting generation of neuronal cells. The present invention provide a composition for preventing or treating a neurological disease, particularly brain disease, and a composition for improving a cognitive function, which comprises stanniocalcin 2 as an active ingredient.

<110>    REGERON, INC.

<120>    COMPOSITIONS FOR PREVENTING OR TREATING BRAIN DISEASE

<130>    PN090048

<150>    KR 10-2008-0008207
<151>    2008-01-25

<160>    11

<170>    KopatentIn 1.71

<210>    1
<211>    25
<212>    DNA
<213>    Homo sapiens

<400>    1
ccggaattca tgtgtgccga gcggc                                          25


<210>    2
<211>    29
<212>    DNA
<213>    Homo sapiens

<400>    2
ggatctccgc tgtattctgc agggacagg                                      29


<210>    3
<211>    28
<212>    DNA
<213>    Homo sapiens

<400>    3
cagaatacag cggagatcca gcactgtt                                       28


<210>    4
<211>    28
<212>    DNA
<213>    Homo sapiens

<400>    4
atgacttgcc ctgggcatca aattttcc                                       28


<210>    5
<211>    30
<212>    DNA
<213>    Homo sapiens

<400>    5
gatgcccagg gcaagtcatt catcaaagac                                     30

<210>	6
<211>	27
<212>	DNA
<213>	Homo sapiens

<400>	6
cacgtagggt tcgtgcagca gcaagtc	27

<210>	7
<211>	27
<212>	DNA
<213>	Homo sapiens

<400>	7
gctgcacgaa ccctacgtgg acctcgt	27

<210>	8
<211>	30
<212>	DNA
<213>	Homo sapiens

<400>	8
ggggtacctc acctccggat atcagaatac	30

<210>	9
<211>	34
<212>	DNA
<213>	Homo sapiens

<400>	9
gtatcagagg tgtctatgac cgacgccacc aacc	34

<210>	10
<211>	30
<212>	DNA
<213>	Homo sapiens

<400>	10
ccggaattcg taaacctctt ccttcaggct	30

<210>	11
<211>	26
<212>	DNA
<213>	Homo sapiens

<400>	11
catagacacc tctgatactc gtttcg	26

## Description

### BACKGROUND OF THE INVENTION

### FIELD OF THE INVENTION

The present invention relates to a composition for preventing or treating a neurological disease and a composition for improving a cognitive function, and more specifically, the composition for preventing or treating a neurological disease (particularly brain disease), and the composition for improving the cognitive function which comprises stanniocalcin 2 as the active ingredient.

### DESCRIPTION OF THE RELATED ART

According to a ratio of aged population announced by Korea National Statistical Office on July 2007, the population over 65 years of age was estimated in 7.3% among total populations in 2000, indicating that the Republic of Korea enters aging society. In 2007, it was estimated as 9.3% increased up to 2.0% compared to 7.3% in 2002. In this connection, it was predicted that an aging index (means a ratio of the person over 65-year old per 100 persons of 0-14 years old) in the Republic of Korea in 2050 will reach at 429 corresponding to 5-fold of global means (82), supposing that it will be the highest value in the world. In addition, it was predicted that a ratio of super-aging population over 80 years of age in 2005 is 1.4% similar with world means (1.3%) whereas increased to 14.5% much higher than the level of advanced country (9.4%) in 2050. Consistent with this tendency, degenerative neurological disorders such as Alzheimer's disease, Huntington's disease, Parkinson's disease and amyotrophic lateral sclerosis (ALS), ischemia such as stroke (particularly, ischemic stroke), or diseases generated by injury of nervous cells caused from reperfusion have been rapidly emerged as a causative factor of death.

A stroke (often called as cerebral apoplexy) is a cerebral disease which results in abnormalities in some cerebral tissue caused by rupture or obstruction of blood vessel in brain, and is a most highly ranked cause of death in the Republic of Korea. As a stroke may develop in any part of the brain, it results in dysfunctions in a body. Medically, a stroke is divided into two types: (a) ischemic stroke caused by obstruction of blood circulation generated in a region according to blockage of blood vessel in brain; and (b) hemorrhagic stroke due to leakage of blood in brain. The incidence rate of ischemic stroke may be higher than hemorrhagic stroke, and ischemic stroke is closely correlated with risk factors such as hypertension or arteriosclerosis known as a cause of adult disease.

Ischemic stroke may be caused by obstruction of any vessel in a body, for example including carotid artery in neck, vertebral basilar artery, thin artery within brain, and so on, leading to 'infarction' which means a death of a brain tissue controlled by these vessels. As no function may be recovered for good at the region at which infarction is developed, central nervous system (CNS) disorders by cerebral infarction would be permanent without recovery. Therefore, the most important thing in treatment of stroke is prevention of cerebral ischemia itself together with prevention of risk factors such as hypertension, diabetes or hyperlipidemia. In treatment of cerebral infarction by stroke development, there have been focused on reduction of secondary brain injuries, for example including decrease of cerebral edema, or suitable promotion of blood flow in ischemic regions.

An example of materials currently used for neuronal protection includes excitatory amino acid antagonists such as ganglioside and nimodipine, and GABA agonists such as clomethiazole. Magnesium sulfate and glycine antagonist are 2^{nd} phase clinical trial, and a large-scale clinical trial is being performed about piracetam. However, the conventional neuroprotective agents were mainly aimed at acting on each step in ischemia development, and thus still remains a need to develop a composite agent acting on many steps at the same time with little side effects and drug complications.

As the symptom of ischemic stroke has been suddenly developed without specific prognosis, it has been considered as much effective method to eat functional foods for constant prevention of ischemia and inhibition of post-ischemic neuronal apoptosis, not to prevent ischemic stroke itself through administering drugs after cerebral ischemia development.

U.S. Patent No. 6,245,757 discloses a use of progestin for treating cell impairment by ischemia, and U.S. Patent No. 6,380,193 discloses a pharmaceutical composition for treating stroke comprising poly(adenosine 5'-diphosphoribose)polymerase inhibitor. In addition, U.S. Patent No. 6,288,041 discloses a pharmaceutical composition for treating stroke comprising sialic acid derivatives, and U.S. Patent No. 5,580,866 discloses a composition for treating stroke comprising 1,4-thiazepines.

Parkinson's disease (PD) is a neurodegenerative disease of the central nervous system that may cause impairment of movement and cognitive function, and was first reported in 1817 by James Parkinson. In US, approximate 750,000-1,000,000 PD patients have been reported at an attack rate of 100-150 persons per 100,000 people, and sixty thousand of new PD patients are added to the list each year. Considering the global trend of aging society, its incidence rate and prevalence rate is also expected to increase in the Republic of Korea. PD has the characteristics inducing: (a) loss of dopamine neuronal cells in substantia nigra; and (b) decrease of dopamine in caudate nucleus and putamen as a projection region of nerve fiber, leading to impairment in movement and cognitive function such as tremor, bradykinesia, rigidity and disturbance of posture.

Major therapeutic drugs to be useful for PD function as follows: (a) to supplement dopamine activity in brain; (b) to prevent or delay destruction of neuronal cells; or (c) to modulate accessory symptoms such as other depression. There have been developed various neuropharmacological reward therapies using representative drugs including: (a) dopamine precursors such as Madopar (levodopa, L-dopa; dopamine precursor); (b) dopamine receptor agonists such as bromidine and lisuride; (c) anti-acetylcholine drugs such as artane; and (d) cogentin. Of them, L-dopa supplements dopamine concentration in brain, contributing to improvement of PD symptoms in most effective manner. However, the administration of L-dopa during the long-term period over 3-5 years results in adverse effects, for example including: (a) wearing-off phenomenon that drug effective time is gradually shortened; (b) on-off phenomenon that the fluctuation of motion control function becomes serious; or (c) abnormal motion symptom (diskinesia) (Freed et. al., N. Engl, J. Med. 327: 1549-55 (1992)).

Further, surgical therapies for PD including thalamotomy, pallidotomy, deep brain stimulation, neuronal cell transplantation have been performed. However, lasting time of treatment efficacy differs significantly from patient to patient along with adverse effects such as hypophonia accompanying operation, dysarthria, a decline in memory, and so on (Ondo et. al., Neurology 50: 266-270 (1998); Shannon et. al., Neurology 50: 434-438 (1998)).

Recent tendency to treat Alzheimer's disease focuses on the fact that Alzheimer's disease may be caused by impaired cholinergic signaling and transmission in cerebral cortex and hippocampus (Bartus et al., Science. 217 (4558): 408-14 (1982)); and Coyle et al., Science. 219 (4589): 1184-90 (1983)). Because this region in brain is related to memory and intelligence, functional defects in this region may induce definite injuries for memory and decision and loss of intelligence. Although the process of impairment in neuronal signaling is still controversial, senile plaque and neurofibrillary tangle (NFT) are considered as major pathological phenotypes in Alzheimer's disease. The notable feature of senile plaque is accumulation of amyloid-β (Aβ), and thus Alzheimer's disease may be confirmed by post-mortem examination (Khachaturian, Arch. Neurol. 42(11): 1097-105(1985)).

Therapeutic drugs and methods for Alzheimer's disease have been suggested as follows: (a) increase of acetylcholine amount to inhibit impairment of cholinergic signaling; (b) long-term maintenance of acetylcholine; or (c) effective acetylcholine activity on signal transmission of neuronal cell. Therefore, there have been utilized a variety of compounds for enhancing acetylcholine activity in patients suffering from Alzheimer's disease. At present, the most efficient approach is to inhibit activity of acetylcholine esterase which blocks neuronal signaling to rapidly decompose acetylcholine in synapse. Practically, these inhibitors (example: tacrine, donepezil, galantamine and rivastigmine) are currently on market as therapeutics for Alzheimer's disease approved by FDA. Despite their effectiveness in preventing further destructive progress of the disease, they are not yet applied to recover patients to pre-illness level.

Some compounds are aimed to improve neuronal condition and maintain aged in good function. For example, a few drugs such as NGF and estrogen play a role in neuron protection by delaying neurodegeneration, and other drugs such as antioxidants reduce cellular oxidation to decrease harmful cell damage caused by oxidation of cells. Alzheimer's disease becomes severe depending on the accumulation extent of amyloid-β peptide. Thus, it could be supposed that the progression of Alzheimer's disease is delayed by lowering the accumulation of amyloid-β in a neuritic space. It also may be assumed that the progression of Alzheimer's disease is varied by diverse combinations between amyloid precursor protein (APP) and cellular proteases such as α-secretase, β-secretase and γ-secretase. However, as amyloid-β formation is not clearly explained in practically scientific manner, it is not yet possible to control amyioid-β formation.

It is not certain how the accumulation of amyloid-β induces acts on neuronal transmission. Abnormal cleaved APP generates massive production of amyioid-β contributing to plaque formation in neuritic space. Therefore, many other factors related with cleavage reaction (example: inflammation response, *etc*.) increase phosphorylation of tau protein and the accumulation paired helical filaments (PHFs) in combination with NFTs, leading to enhance neuronal injury. All these factors generate neuronal impairment and finally expedition of senile dementia of the Alzheimer's type.

Though therapeutic methods to alleviate influence of Alzheimer's disease have been enormously developed, current methods provide only temporary improvement of symptoms. Consequently, treatment of Alzheimer's disease is just focused on improvement of the symptom instead of restoring disease process. Biologically, Alzheimer's disease is well known, but the clinical applications thereof are not yet successful.

U.S. Patent No. 5,532,219 discloses a pharmaceutical composition for treating Alzheimer's disease comprising 4,4'-diaminodiphenylsulfone, and U.S. Patent No. 5,506,097 discloses a pharmaceutical composition for treating Alzheimer's disease comprising para-aminodiphenylmethansulfonyl fluoride or ebelactone A. In addition, U.S. Patent No. 6,136,861 discloses a pharmaceutical composition for treating Alzheimer's disease comprising bicyclo[2.2.1]heptane.

Meanwhile, stress is becoming major problem in health in modern society. It is reported that 1/3 of the twenties in the Republic of Korea usually experience much stresses and that the women suffer from stress than men in their teens, and depending on age. Strength of stress may be varied depending on personality, interest, means of relief from stress, surrounding environment, controlling ability of a person, and most stresses is usually followed by depression. Depression is a mental disorder capable of being developed by stress to generate extreme result such as suicide. Dementia also has been understood as very crucial disorder due to its high rate of occurrence and recurrence. Depression has been reported to be caused by impairment of neurotransmitters including adrenaline, dopamine or serotonin, and followed by cerebral impairment such as atrophy of hippocampus and inhibition of mature neuron formation. Tricyclic antidepressant (TCA) as a representative therapeutic agent for depression has drawbacks of having severe side-effects. Most of all, depression therapeutics including amitriptyline have been commonly prescribed in domestic, resulting in many problems (e.g., various adverse effects). Fluoxetine as selective serotonin re-uptake inhibitor (SSRI) was developed in US in 1980's and had advantages such as: (a) remarkable drug compliance to overcome side-effects of TCAs; and (b) low treatment failure rate. Thus, fluoxetine was ranked in seventh among 20 international drugs in 1996. However, it is problematic that SSRI shows little improvement in efficacy compared with TCA, and still has serious drug interference.

Further, neuronal disturbance is still induced by stress, and there is no way to inhibit the relapse after medicinal treatment of depression, and thus a long-term administration with a lowered dose is only used at present. Therefore, it is very important to develop a substance which has superior effect on inhibition of neuronal apoptosis or correction of neurotransmitter system caused by stress. In addition, it is very necessary to develop a functional food having a therapeutic potential for stress-induced depression as considering a tendency of avoiding visiting treatment care institutions, or overlooking the induction of disturbance in serotonin neuronal system and cerebral impairment.

There have been enormously attempted in worldwide country for prevention of neurodegenerative diseases and development of their therapeutics. As a representative, U.S. Patent No. 6,020,127 discloses a gene encoding a neuronal apoptosis inhibitor protein in human chromosome 5q13, U.S. Patent No. 6,288,089 discloses pyridyl imidazoles for treating neurodegenerative disorders by inhibiting apoptosis of dopamine neurons.

Presently, ischemia-reperfusion method is suggested as an experimental method which enables to similarly reproduce the mechanism of brain impairment induced by the above-mentioned various causes. A mechanism of neuronal damage generated by this method may be widely summarized as two hypotheses: (a) excitotoxicity hypothesis that neuronal cells are killed by allowing high levels of calcium ions (Ca²⁺) to be entered into the cell via opening of calcium ion channel depending on excess increase of glutamate after ischemia-reperfusion (Hagberg, H. et al., Ment Retard Dev Disabil Res Rev. 8 (1): 30-38 (2002)); and (b) oxidative stress hypothesis that neuronal death is derived from destruction of cellular enzymes by reperfusion-induced free radicals (Chan. PH., J Cereb Blood Flow Metab. 21 (1): 2-14 (2001)). According to the research, it has been known that neuronal death by both excitotoxicity and oxygen radicals is generated not independently but simultaneously, and functions directly (Won, MH. et al., Brain Res. 836: 70-78 (1999)). Recently, there has been focused on a substance such as inflammsatory mediators or cytokines induced at four day when cell death is generated. It is known that these inflammatory mediators or cytokines are secreted from microglia or dying cells to increase cell death (Basu, A. et al., J Neurosci. 22: 6071-6082 (2002)).

In the senses that excitotoxicity by excitatory amino acids (kainic acid, NMDA, quisulate, AMPA, glutamate, etc.) allows neuronal death and disorder of brain function to generate various brain disorders such as stroke, Alzheimer's disease, Parkinson's disease and spinal cord injury, alternative experimental method related to the mechanism of brain injury is a method to inject kainic acid into ventricle. Where kainic acid is administered into systemic or central nervous system, epilepsy seizure is increased and the expression of prodynorphin or proenkephalin mRNA synthesizing opioid peptide is enhanced in hippocampus. Kainic acid induce not only the expression of c-fos and c-jun transcription factor in hippocampus (Kaminska, B. et al., Acta Biochim Pol. 44: 781-789 (1997)), but also the expression of cytokine gene such as IL-1, and iNOS (inducible nitric oxide synthase) and cyclooxygenase-2 (COX-2) gene related with inflammation response (Kunz, T. and Oliw, EH., Eur J Neurosci. 13: 569-575 (2001)). In MAPK signal transduction study using kainic acid in white mice, it is known that phosphorylation of ERK1/2, p38 or JNK protein are enhanced in hippocampus, and kainic acid-induced MAPK expression is an important indicative to induce expression of many genes downstream in cell survival or death (Mielke, K et al., Neuroscience. 91: 471-483 (1999)).

Throughout this application, various patents and publications are referenced and citations are provided in parentheses. The disclosure of these patents and publications in their entities are hereby incorporated by references into this application in order to more fully describe this invention and the state of the art to which this invention pertains.

### DETAILED DESCRIPTION OF THIS INVETNION

### Technical Problems to be Solved

The present inventors have made intensive researches to develop novel therapeutics for preventing or treating brain diseases and improving cognitive functions by inhibiting apoptosis of neuronal cells and promoting generation of neuronal cells. As a result, we have discovered that stanniocalcin 2 has the activities described above for neuronal cells.

Accordingly, it is one object of this invention to provide a composition for preventing or treating a brain disease, which comprises stanniocalcin 2 as an active ingredient.

It is another object of this invention to provide a composition for improving a cognitive function, which comprises stanniocalcin 2 as an active ingredient.

It is still another object of this invention to provide a method for preventing or treating a brain disease.

It is further object of this invention to provide a method for improving a cognitive function.

Other objects and advantages of the present invention will become apparent from the detailed description to follow taken in conjugation with the appended claims and drawings.

### Technical Solutions for Overcoming Technical Problems

In one aspect of this invention, there is provided a composition for preventing or treating a brain disease, which comprises stanniocalcin 2 as an active ingredient.

In another aspect of this invention, there is provided a composition for improving a cognitive function, which comprises stanniocalcin 2 as an active ingredient.

In still another aspect of this invention, there is provided a method for preventing or treating a brain disease, which comprises administering to a subject a pharmaceutical composition comprising stanniocalcin 2 as an active ingredient.

In further aspect of this invention, there is provided a method for improving a cognitive function, which comprises administering to a subject a pharmaceutical composition comprising stanniocalcin 2 as an active ingredient.

The present inventors have made intensive researches to develop novel therapeutics for preventing or treating brain diseases and improving cognitive functions by inhibiting apoptosis of neuronal cells and promoting generation of neuronal cells. As a result, we have discovered that stanniocalcin 2 has the activities described above for neuronal cells.

Unlikely to stanniocalcin 1, little has been yet known about physiological functions of stanniocalcin 2 used as active ingredients in the present invention. Stanniocalcin 2 has been only anticipated to have different roles from stanniocalcin 1 (Derek A. JELLINEK et al., Biochemical J., 350: 453-461 (2000)).

Stanniocalcin (STC) is a homodimeric glycoprotein hormone that was first discovered in fish, where it is produced by unique endocrine glands known as the corpuscles of Stannius (Wagner, G., Biochemistry and Molecular Biology of Fishes, eds. Hochachka and Mommsen, Elsevier Science, Amersterdam, 2 (21): 419-434 (1993)). Human stanniocalcin has been recently discovered (Chang et al., Mol. Cell. Endocrinol. 112: 241-247 (1995) and Olsen et al. WO 95/24411). Little has been yet known about physiological functions of stanniocalcin 2 used as active ingredients in the present invention. Stanniocalcin 2 (STC2) has been only anticipated to have different roles from stanniocalcin 1 (STC1) (Derek A. JELLINEK et al., Biochemical J., 350: 453-461 (2000)). Stanniocalcin 2 has a sequence homology of about 34% to STC1 (Chang, A.C.M. et al., Mol. Cell. Endocrinol., 141: 95-99 (1998)). STC2 is widely expressed in mammalian tissues such as pancreas, skeletal muscle and small intestine. STC2 has been expected to play a role in calcium and phosphate metabolism, although not well verified.

The most striking feature of the present invention resides on our novel findings in which STC2 inhibits neuronal death and promotes generation of neuronal cells.

The composition of this invention comprising STC2 is very effective in preventing or treating a variety of neurologic diseases, *inter alia,* brain diseases. The therapeutic effects of the present composition are ascribed to its neuroprotective actions. The term used herein "neuronal cell" refers to central nervous system, brain, brainstem, spinal cord, neuron having a structure connecing central nervous system and peripheral nervous system, and neuronal supporting cell, Glia and Schwann cell. As used herein, the term "protective activity for neuronal cell" refers to the effect of reducing or ameliorating neurologic insult, and protecting or reviving neuronal cell that has suffered neurologic insult. In addition, the term "neurologic insult" used herein means any damage to neuronal cell or tissue resulting from various causes such as metabolic, toxic, neurotoxic and chemical causes.

A Practical example of disease or disorder applicable to the composition of the present invention includes, but not limited to, a neurodegenerative disease, an ischemia-reperfusion injury and a mental disorder. More specifically, the composition of the present invention may be utilized as a composition for preventing or treating a neurodegenerative disease such as Alzheimer's disease, Huntington's disease, Parkinson's disease and amyotrophic lateral sclerosis (ALS); ischemia or reperfusion injury such as stroke (particularly, ischemic stroke); and mental disorder such as schizophrenia, depression, manic depression and post traumatic stress disorder.

As shown in Examples below, stanniocalcin 2 (STC2) of the present invention may remarkably inhibit a cell death via a neuronal apoptosis. For example, STC2 may significantly inhibit neuronal death by kainic acid as a neurotoxic substance which induces a neuronal apoptosis.

Stanniocalcin 2 (STC2) of the present invention may strikingly improve a cognitive function. Preferably, STC2 of the present invention has a superior activity for improving or preventing impairment of cognitive function caused by the above-described neurological diseases. In addition, STC2 of the present invention has an excellent efficacy on improvement of cognitive function in the normal person.

Meanwhile, hippocampus of the brain is the most important region in the formation and storage of memory. Hippocampus is a neuron-dense region in which new neuronal cells is actively produced and is responsible for learning and memory function via reciprocal electrical stimulation. STC2 of the present invention promoted neurogenesis, particular in subgranular zone (SGZ) beneath granular cell layer (GCL) of dentate gyrus (DG) inside hippocampus. Given that imipramine as a representative antidepressant has no effect on treatment of depression where neurogenesis in hippocampus is not generated, neurogenesis in GCL of hippocampal DG may be associated with improvement of stress. As another antidepressant paroxetine is known to promote neurogenesis in GCL of hippocampal DG, it is preferable that stanniocalcin 2 is utilized as a therapeutic agent against depression.

According to a preferable embodiment, STC2 of the present invention has an activity for improvement of cognitive function, for example including improvement of learning ability and/or memory.

The term "prevention" used herein refers to inhibiting the generation of disorders or diseases in animal who are not diagnosed to have but are susceptible to such disorders or diseases. As used herein, the term "treatment" refers to (a) inhibiting the development of disorders or diseases; or (b) ameliorating or (c) removing the disorders or diseases.

The term "stanniocalcin 2" used herein refers to human stanniocalcin 2 unless otherwise indicated, and preferably an amino acid sequence of SEQ ID NO:1.

According to a preferable embodiment, the composition of this invention is pharmaceutical composition or a food composition.

The pharmaceutical composition of this invention includes (a) a therapeutically effective amount of stanniocalcin 2; and (b) a pharmaceutically acceptable carrier.

In the pharmaceutical compositions of this invention, the pharmaceutically acceptable carrier may be conventional one for formulation, including carbohydrates *(e.g.,* lactose, amylase, dextrose, sucrose, sorbitol, mannitol, starch, cellulose), acacia rubber, calcium phosphate, alginate, gelatin, calcium silicate, fine crystallite cellulose, polyvinylpyrrolidone, cellulose, water, syrups, salt solution, alcohol, Arabian rubber, vegetable oil (e.g., corn oil, cotton seed oil, soybean oil, olive oil and coconut oil), poly(ethylene glycol), methyl cellulose, methylhydroxy benzoate, propylhydroxy benzoate, talc, magnesium stearate, and mineral oils, but not limited to. The pharmaceutical composition according to the present invention may further include a lubricant, a humectant, a sweetener, a flavoring agent, an emulsifier, a suspending agent, and a preservative, but not limited to. Details of suitable pharmaceutically acceptable carriers and formulations can be found in Remington's Pharmaceutical Sciences (19th ed., 1995), which is incorporated herein by reference.

The pharmaceutical composition according to the present invention may be administered via the oral or parenterally. When the pharmaceutical composition of the present invention is administered parenterally, it can be done by intravenous, subcutaneous, intramuscular and intracerebroventricular administration.

A suitable dose of the pharmaceutical composition of the present invention may vary depending on pharmaceutical formulation methods, administration methods, the patient's age, body weight, sex, severity of diseases, diet, administration time, administration route, an excretion rate and sensitivity for a used pharmaceutical composition. Physicians with average skill may easily determine and diagnose dosage level of medicine effective for treating or preventing target disorders or diseases. Preferably, the pharmaceutical composition of the present invention is administered with a daily dose of 0.0001-100 mg/kg (body weight).

According to the conventional techniques known to those skilled in the art, the pharmaceutical composition may be formulated with pharmaceutically acceptable carrier and/or vehicle as described above, finally providing several forms including a unit dose form and a multi-dose form. Formulation may be oil or aqueous media, resuspension or emulsion, extract, powder, granule, tablet and capsule and further comprise dispersant or stabilizer.

The composition of the present invention may be provided as a food composition, particularly a functional food composition. The functional food composition of the present invention may be formulated in a wide variety of forms, for example, including proteins, carbohydrates, fatty acids, nutrients, seasoning agents and flavoring agents. As described above, an example of carbohydrate may include monosaccharides (*e. g.,* glucose, fructose, *etc.*); disaccharides (*e. g.,* maltose, sucrose, *etc*.); oligosaccharides; polysaccharides (*e. g.,* common sugars including dextrin, cyclodextrin, *etc*.); and sugar alcohols (*e. g.,* xylitol, sorbitol, erythritol, *etc*.). The formulation of flavoring agent may use natural flavoring agents (*e. g.,* thaumatin, stevia extract (e.g., rebaudioside A, glycyrrhizin), *etc*.) and synthetic flavoring agents (*e. g.,* saccharine, aspartame, *etc*.). In the formulation of drinking agent, it may further include citric acid, liquid fructose, sweet, glucose, acetic acid, malic acid, fruit syrup, eucommia bark extract, jujube extract and glycyrrhiza extract. Considering easy accessibility of food, the food composition herein is very useful in prevention or treatment of brain disorders or diseases, or improvement of cognitive function.

The features and advantages of the present invention will be summarized as follows:
(i) The present invention provide a composition for preventing or treating a neurological disease, particularly brain disease, and a composition for improving a cognitive function, which comprises stanniocalcin 2 as an active ingredient.
(ii) Stanniocalcin 2 as the active ingredient of the present invention has a superior activity for inhibiting neuronal apoptosis, and interestingly promoting neurogenesis.

### BRIEF DESCRIPTION OF THE DRAWINGS

Fig. 1 represents that stanniocalcin 2 (STC2) prevents neuronal death in cornu Ammonis 3 (CA3) of mouse hippocampus. To compare KA alone with KA and STC2, panel A and C are treated with kainic acid (KA) alone, and panel B and D are treated with both KA and STC2. In panel A, each CA1, CA2 and CA3 indicates cornu Ammonis (CA) field 1, field 2 and field 3 of hippocampus, and DG indicates dentate gyrus (DG). In panel C, three block arrows represent an apoptotic region of neuronal cell. In panel B and D, it was demonstrated that STC2 enables to inhibit neuronal death.
Fig. 2 represents genome of postmitotic neurons stained with bromodeoxyuridine (BrdU) using immnohistochemistry to examine STC2 effects on neuron proliferation in subgranular zone (SGZ) located in mouse hippocampus. Black arrows indicate BrdU-immunopositive cells. It could be demonstrated that BrdU-immunopositive cells in panel B and D (STC2) are increased in SGZ compared to panel A and C (control).
Fig. 3 is a comparative graph relatively quantifying experimental results of Fig. 2. Control is a group without STC2, and STC2 is a group with STC2. It could be appreciated that BrdU-immunopositive cells in SGZ are significantly enhanced in STC2-treated group compared with control.
Fig. 4 shows SDS-PAGE on precipitates centrifuged after Top10F' cells transformed with pUC-narK Met-hSTC2 are homogenized, indicating 33 kDa band corresponding to hSTC2.
Fig. 5 is SDS-PAGE of purified hSTC2, indicating 33 kDa band of purified hSTC2.
Fig. 6 is is SDS-PAGE of purified hSTC2, indicating 33 kDa band of purified hSTC2. Lane 1 is protein size marker; and lane 2 is purified hSTC2.
Fig. 7 represents Y-maze behavior in mouse, demonstrating that the score of place memory in hSTC2-treated group is significantly increased compared to KA alone-treated group (p < 0.05).
Fig. 8 is a water finding test in mouse and drinking latency in hSTC2-treated group is significantly reduced compared to KA alone-treated group, meaning excellent learning memory (p < 0.05).
Fig. 9 is a forced swim test in mouse and immobile time in hSTC2-treated group is significantly reduced compared to PB-treated group, representing effective efficacy on improvement of depression-related behavior (p < 0.05).
Fig. 10 represent the extent of brain impair in hSTC2-treated group is significantly reduced in mouse transient focal ischemic model compared to that in PB-treated group, demonstrating effective reduction in volume of cerebral infraction and neurological deficit (p < 0.05).

The present invention will now be described in further detail by examples. It would be obvious to those skilled in the art that these examples are intended to be more concretely illustrative and the scope of the present invention as set forth in the appended claims is not limited to or by the examples.

### EXAMPLES

### Materials and Methods

### 1. Stanniocalcin 2 (STC2) Preparation

Genomic DNA was extracted from HEF (Human embryonic fibroblast) and used as template aftter cutting with *Bam*HI (Takara, Japan). PCR was carried out to obtain four DNA fragments for exon encoding stanniocalcin 2. To ligate exon DNA fragments, primers were designed for base pairing between 19 bases in a linking region, and PrimeSTAR^{™} HS DNA polymerase (Takara, Japan) was used in all PCR reactions. To amplify exon 1, 2, 3 and 4 of stanniocalcin 2, the first PCR method is as follows: (a) genomic DNA cut with *Bam*HI (Takara, Japan) was commonly used as a template; and (b) PCR cycle (98°C 10 sec; 55°C 5 sec; and 72°C 30 sec) using hSTC2 1U primer (Bioneer, Korea) and hSTC2 2D primer, obtaining 169 bp exon 1 fragment. According to the method as described above, 163 bp exon 2, 231 bp exon 3, and 420 exon 4 were obtained using hSTC2 3U primer and hSTC2 4D primer, hSTC2 5U primer and hSTC2 6D primer, and hSTC2 7U primer and hSTC2 8D primer, respectively.

Second PCR utilized exon 1 (169 bp), exon 2 (163 bp), exon 3 (231 bp) and exon 4 (420 bp) obtained by the above-described method as a template, and PCR using hSTC2 1U containing *Eco*RI (Takara, Japan) restriction site (GAATTC) and hSTC2 8D containing *Kpn*I restriction site (GGTACC) was carried out for 30 cycles of 98°C 10 sec; 55°C 5 min; and 72°C 1 min to obtain 926 bp stanniocalcin 2.

The resulting DNA encoding stanniocalcin 2 and pUC-18 (Amersham Pharmacia Biotech, Swiss) was restricted with *Eco*RI and *Kpn*I (Takara, Japan), and ligated with T4 DNA ligase (Takara, Japan), followed by transformation into Top10F' *E. coli.* After incubating at 37°C for 15 hrs, three colonies randomly selected were cultured and plasmids were obtained ccording to alkaline lysis method. These plasmids were electrophoresized on 1% agarose gel and then desirable plasmid (pUC-hSTC2) was selected by analysis using nucleotide sequence kit (Solgent, Korea).

PCR was carried out to link Met-stanniocalcin 2 in which narK promoter and signal sequence are removed, and primers were designed for base pairing between 18 bases in a linking region. The first PCR method is as follows: (a) pNKmut plasmid (-10 mutated narK promoter; Regeron Inc.) was commonly used as a template; and (b) PCR cycle (98°C 10 sec; 55°C 5 sec; and 72°C 25 sec) using OY-17 and r-narK D primer pair, obtaining 350 bp narK promoter. PCR (30 cycles: 98°C 10 sec; 55°C 5 sec; and 72°C 55 sec) was carried out using pUC-hSTC2 as a template and hSTC2 9U and hSTC2 8D primer pair to obtain 863 bp Met-stanniocalcin 2.

Second PCR utilized narK promoter (350 bp) and Met-stanniocalcin 2 (420 bp) obtained by the above-described method as a template, and PCR using OY-17 containing *Eco*RI (Takara, Japan) restriction site (GAATTC) and hSTC2 8D containing *Kpn*I restriction site (GGTACC) was carried out for 30 cycles of 98°C 10 sec; 55°C 5 min; and 72°C 1 min to obtain 1,195 bp fragments containing Met-stanniocalcin 2 which narK promoter and signal sequence is removed. 1,195 bp fragments (containing Met-stanniocalcin 2 which narK promoter and signal sequence is removed) and pUC-rrnB (rrnB terminator is inserted into pUC18; Regeron Inc.) were restricted with *Eco*RI and *Kpn*I, and ligated with T4 DNA ligase, followed by transformation into Top10F' *E. coli.* After incubating at 37°C for 15 hrs, three colonies randomly selected were cultured and plasmids were obtained ccording to alkaline lysis method. These plasmids were electrophoresized on 1% agarose gel and then desirable plasmid (pUC-narK Met-hSTC2) was selected by analysis using nucleotide sequence kit.

**TABLE. Primer nucleotide sequences.**

| Primer | Nucelotide sequence (5'→3') |
|---|---|
| hSTC2 1U | CCGGAATTCATGTGTGCCGAGCGGC (25mer) |
| hSTC2 2D | GGATCTCCGCTGTATTCTGCAGGGACAGG (29mer) |
| hSTC2 3U | CAGAATACAGCGGAGATCCAGCACTGTT (28mer) |
| hSTC2 4D | ATGACTTGCCCTGGGCATCAAATTTTCC (28mer) |
| hSTC2 5U | GATGCCCAGGGCAAGTCATTCATCAAAGAC (30mer) |
| hSTC2 6D | CACGTAGGGTTCGTGCAGCAGCAAGTC (27mer) |
| hSTC2 7U | GCTGCACGAACCCTACGTGGACCTCGT (27mer) |
| hSTC2 8D | GGGGTACCTCACCTCCGGATATCAGAATAC (30mer) |
| hSTC2 9U | GTATCAGAGGTGTCTATGACCGACGCCACCAACC (34mer) |
| OY-17 | CCGGAATTCGTAAACCTCTTCCTTCAGGCT (30mer) |
| r-narK D | CATAGACACCTCTGATACTCGTTTCG (26mer)] |

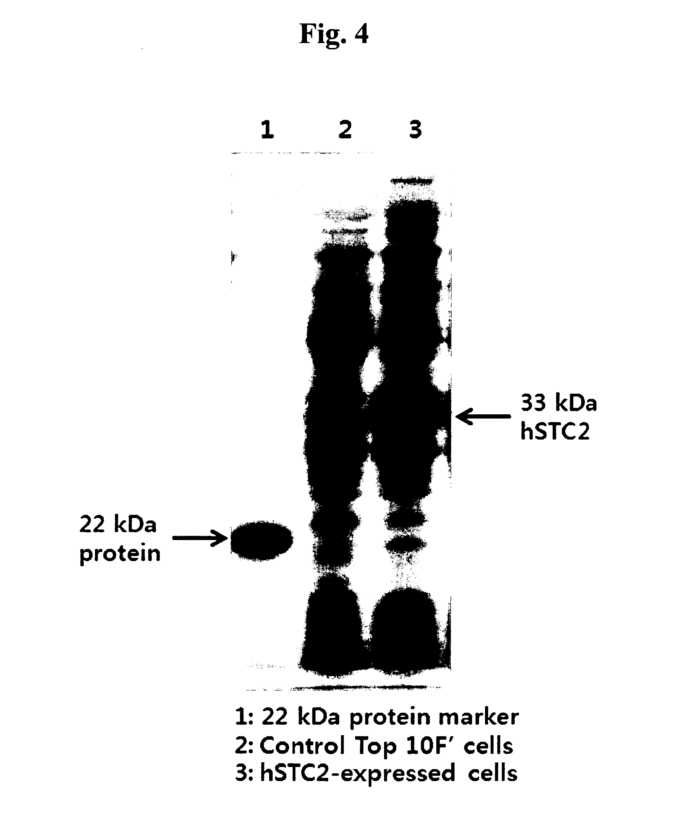

Ten g of Top10F' cells transformed with pUC-narK Met-hSTC2 was suspended in 200 ml of 50 mM EDTA solution, and then sonicated, followed by centrifuging at 10,000 g for 30 min to collect precipitates. The precipitates were resuspended and then analyzed on SDS-PAGE. As shown in Fig. 4, about 33 kDa band indicating hSTC2 was observed. In addition, 33 kDa band on SDS-PAGE was eluted and incubated with trypsin (Promega, US) at 37°C for 16 hrs. As a result, it could be demonstrated that the band is hSTC2 using MALDI-TOF (Applied Biosystems, US) and MS-Fit search (Protein Prospector).

The centrifuged precipitates were mixed to 200 ml distilled water, and 1 ml of 100% Triton X-100 was added to a concentration of 0.5%, followed by shaking at room temperature for 30 min. The precipitates were harvested by centrifuging at 10,000 g for 30 min. The precipitates were dissolved in 200 ml distilled water, and stirred at room temperature for 30 min. The precipitates were collected by centrifuging at 10,000 g for 30 min. After the precipitates were mixed with solution A (50 mM Tris pH 8.0, 6 M Urea, 10 mM 2-Mercaptoethanol), the mixture was stirred at room temperature for 90 min, and centrifuged at 10,000 g for 40 min, obtaining the supernatant.

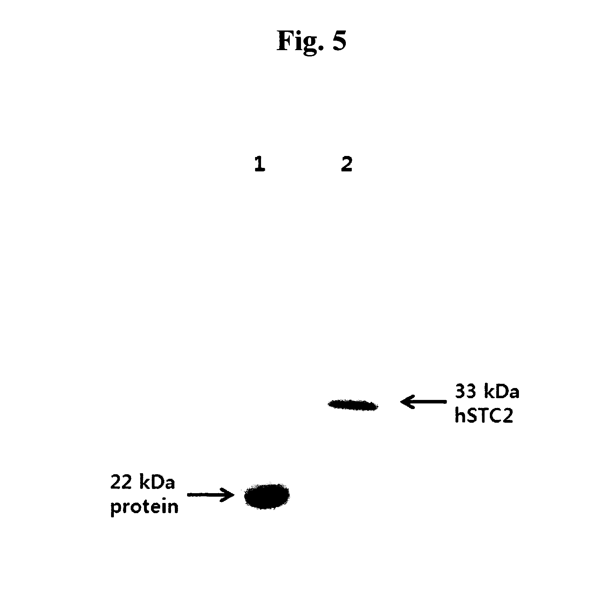

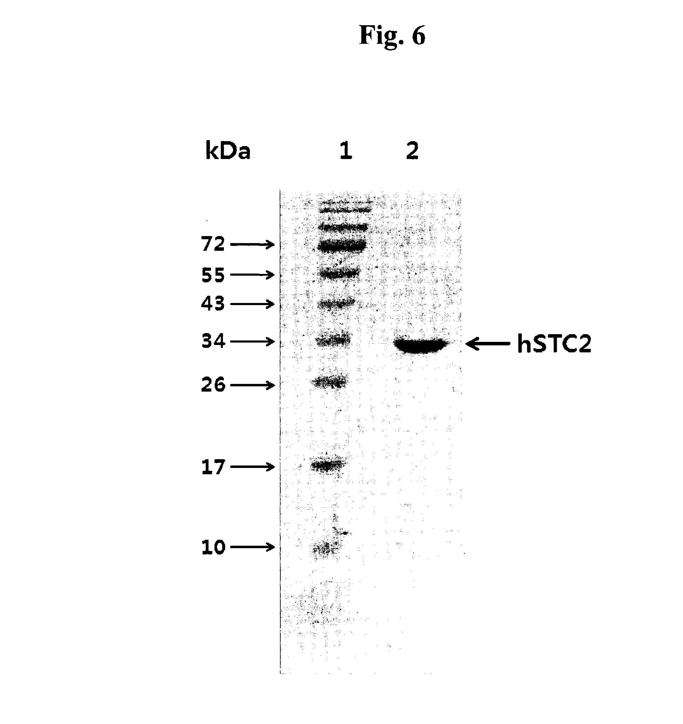

The supernatant was diluted with 200 ml distilled water, and adsorbed to gel by passing DEAE-Sepharose column (GE Healthcare) pre-equilibrated with a buffer solution (20 mM Tris, 1 mM EDTA), followed by washing with the buffer solution (20 mM Tris, 1 mM EDTA). The adsorbed proteins were eluted from the gel using a buffer solution (20 mM Tris, 1 mM EDTA, 300 mM NaCl). The eluent is subjected to gel filtration chromatography using Superdex 200 (GE Healthcare) pre-equilibrated with a buffer solution (20 mM NaH₂PO₄, 1 mM EDTA, pH 7.0). The eluted fractions were electrophoresized on 15% SDS-PAGE (Fig. 5) to collect only the fractions which hSTC2 purity is higher than 90%. Finally, purified hSTC2 (not less than 90% purity; Fig. 6) was measured at 595 nm using Bradford assay with standard protein (BSA; bovine serum albumin) and Spectra MAX 190 (Molecular Device Inc.), obtaining quantitative protein amount of 0.125 mg/ml. Final purified hSTC2 was utilized in further experiments.

### 2. Determination of Stanniocalcin 2 (STC2) Concentration for Intracerebroventricular (I.C.V) Injection

Five µl hSTC2 (100 ng/ 5 µl in PBS) was intracerebroventricularly injected to ICR mouse (DBL, Korea).

### 3. Intraperitoneal injection of Bromodeoxyuridine (BndU)

Fifty mg/kg BrdU was intraperitoneally injected to four-week-old male ICR mouse (DBL, Korea) with weight of 23-25 g.

### 4. Bromodeoxyuridine (BrdU) Staining

hSTC2 was intracerebroventricularly injected to four-week-old male ICR mouse (DBL, Korea) with weight of 23-25 g and then BrdU was intraperitoneally injected. After injection for 24 hrs, experimental animals were subjected to perfusion fixation using 4% paraformaldehyde preperfusion solution. Afterward, brain was immediately extracted from the animals and was washed with 30% sucrose solution for 24 hrs after postfixation in equal solution for 4 hrs. The brain tissues were frozen using optimum cutting temperature compound (OCT compound, Fisher). Tissue sections with 40 µm thickness was prepared using a freezing microtome and added with cryoprotectant solution, followed by being stored at -20°C for BrdU staining. Experiments were carried out for 2 days. In first day, brain tissues immersed in cryoprotectant solution were transferred to acryl plate well, and washed three times with 50 mM phosphate buffer (PB) for 5 min. After treatment with 0.5% Triton X-100 for 20 min, the tissues were washed three times with 50 mM phosphate buffer (PB) for 5 min, and transferred into glass bottle containing 2 ml solution which is composed of 50% formamide and 2xSSC prepared using 100% formamide and 4xSSC, followed by incubating at 65°C for 2 hrs in a shaking incubator. After washing with 2xSSC two times for 5 min, the tissues were added with 2 N HCl (9.6 ml PBS + 2 ml HCl conc.) prewarmed at 37°C for 30 min in a shaking incubation bath, and neutralized at 25°C for 10 min in 0.1 M sodium borate (pH 8.5) with shaking. The tissues were washed three times with 50 mM PB for 5 min, and incubated with 1% BSA (bovine serum albumin) and 10% horse serum for 1 hr, followed by immunohistochemical staining at 4°C for 12 hrs using anti-BrdU antibody (Roche). Next day, the brain tissues were washed three times with 50 mM PB for 5 min, and incubated with biotin-conjugated goat anti-mouse IgG secondary antibody (1:200, Vector) contained in 50 mM PB and 0.5% BSA for 1 hr, followed by washing three times with 50 mM PB for 5 min. The tissues were incubated with ABC (avidin-biotin complex) reagent (1:200, Vector) for 1 hr, and washed three times with 50 mM PB for 5 min, followed by colorimetric reaction using diaminobenzidine (DAB) as a substrate. After stopping reaction, the tissues were stained with cresyl violet for about 2 min, and had transparent by dehydration using conventional methods. Finally, the tissues were embedded in polymount.

### 5. Immunohistochemistry

Five µl of kainic acid (0.1 µg/5 µl, Tocoris), or 5 µl of mixture solution (containing 0.1 µg kainic acid and 100 ng hSTC2 in 5 µl solution) was intracerebroventricularly (I.C.V) injected to four-week-old male mouse with weight of 23-25 g. After injection for 24 hrs, experimental animals were subjected to perfusion fixation using 4% paraformaldehyde preperfusion solution. Afterward, brain was immediately extracted from the animals and was washed with 30% sucrose solution for 24 hrs after postfixation in equal solution for 4 hrs. The brain tissues were frozen using OCT compounds. Tissue sections with 40 µm thickness was prepared using a freezing microtome and added with cryoprotectant solution, followed by being stored at -20°C for immunohistochemistry. In first experimental day, brain tissues immersed in cryoprotectant solution were washed three times with 50 mM PB for 5 min. The tissues was treated with 3% H₂O₂ (in 50 mM PB) for 10 min to remove endogenous peroxidase, and incubated with 50 mM PB, 1% BSA and 0.2% Triton X-100 for 30 min. After incubating with 50 mM PB, 0.5% BSA and 3% normal serum for 1 hr, the tissues were washed with 50 mM PB for 10 min, and immunohistochemically stained with using anti-OX-42 monoclonal antibody. Next day, the brain tissues were washed three times with 50 mM PB for 5 min, and incubated with goat anti-mouse IgG secondary antibody (1:200) contained in 50 mM PB and 0.5% BSA for 1 hr, followed by washing three times with 50 mM PB for 5 min. The tissues were incubated with ABC reagent (1:200) for 1 hr, and washed three times with 50 mM PB for 5 min, followed by colorimetric reaction using DAB as a substrate. After stopping reaction, the tissues had transparent by dehydration using conventional methods and finally embedded in polymount.

### 6. BV2 microglia culture

Mouse microglia cell line, BV2 (kindly provided by Dr. Cho dong-hyup, Department of Neurobiology and Behavior, Cornell University), was cultured in DMEM (Dulbeco's Modified Eagle's Medium) media (GIBCO BRL, USA) supplemented with 2 mM L-glutamine, 100 U/ml penicillin, 100 µg/ml streptomycin, 10% heat-inactivated fetal bovine serum (FBS) at 37°C in 5% CO₂ incubator. Cells were subcultured when they were grown to about 90% of bottom area, and cells of exponential growth phase were used for further experiments.

### 7. Drug Treatment

To inhibit microglia activity, hSTC2 was treated at a final concentration of 10 nM. As a microglia activator, LPS (lipopolysaccharide) was treated at a final concentration of 200 ng/ml.

### 8. Measurement of Nitric Oxide (NO) Concentration

The production of nitric oxide (NO) was determined by measuring concentration of nitrite (NO₂⁻). The concentration of nitrite was measured by colorimetric assay using Griess reagent (1% sulfanilamide, 0.1% naphthyl-ethylenediamine dihydrochloride/2.5% H₃PO₄).

### 9. Mouse Y-maze Test

Four-week-old male ICR mouse (DBL, Korea) with weight of 23-25 g was randomly divided into two groups (control and test), and each group consists of five mice. Five µl of kainic acid (0.1 µg/5 µl, Tocoris) and 5 µl of mixture solution (containing 0.1 µg kainic acid and 100 ng hSTC2 in 5 µl solution) were intracerebroventricularly (I.C.V) injected to control and test group, respectively. After injection for 24 hrs, Y-maze experiment was performed to examine cognitive function. Y-maze device is composed of three arms with 40 (width) x 12 (length) x 30 (height), and experiment was carried out in intensity of illumination of 20 ± 5 lux. Each three arms consisting of Y-maze was randomly named as A, B and C. After a head part of mouse was put toward the passage in the end of an arm, mouse wandered into the passage in a free manner for 8 min to observe movement path. Passing of the arm on Y-maze of this invention means that hind legs of mouse are entered into the passage of an arm. As described above, arms that mouse passes were sequentially recorded and then tied up three in a sequence. As a result, it was considered as one point that all paths (arms) is independently different, which mouse passes. For example, where mouse passes the arm in a sequence of ABCAC, the order of ABC, BCA and CAC is tied, giving two points. Memory score (%) is calculated as follows: total score is divided by (total path number-2) and converted to percentage.

### 10. Mouse Water Finding Test

Four-week-old male ICR mouse (DBL, Korea) with weight of 23-25 g was randomly divided into two groups (control and test), and each group consists of five mice. Five µl of kainic acid (0.1 µg/5 µl, Tocoris) and 5 µl of mixture solution (containing 0.1 µg kainic acid and 100 ng hSTC2 in 5 µl solution) were intracerebroventricularly (I.C.V) injected to control and test group, respectively. After injection for 24 hrs, water finding test was performed to suppose latent learning. A device was a box in a size of 30 (width) x 50 (length) x 20 (height), and its bottom was divided into 15 spaces of 10 x 10 cm, of which a door of 10 x 10 cm was prepared one wall, and a water bottle was put inside the door. In first day, mouse injected with kainic acid alone or kainic acid and STC2 was placed in one end of the space and learned to drink water. After learning, the supply of water was stopped for 24 hrs. In second day, the mouse was again put into the device, and then the time (sec) of drinking latency was measured.

### 11. Mouse Forced Swim Test

Four-week-old male ICR mouse (DBL, Korea) with weight of 23-25 g was randomly divided into two groups (control and test), and each group consists of five mice. Five µl of PBS and 5 µl of hSTC2 (100 ng/5 µl) were intracerebroventricularly (I.C.V) injected to control and test group, respectively. After injection for 24 hrs, immobilization stress was forced for 2 hrs. After stress, the mouse was subjected to forced swim for 6 min in circular water bath (diameter, 10 cm; height, 20 cm) containing water of 25 ± 2°C. Two min later, the time in an immobile floating posture that the face of mouse is floated on the surface of the water was measured for 4 min. An immobile behavior is known to be helplessness.

### 12. Effects on Brain Impair Caused by Transient Focal Cerebral Ischemia and Middle Cerebral Artery Occlusion (MCAO)

Ten of adult male C57BL/6J mice (3-month-old, 25-30 g, DBL, Korea) were anesthetized by intraperitoneal injection with tiletamine, zoletile and xylazine hydrochloride (8 mg/kg), and immobilized on stereotaxic instrument (Harvard Apparatus). After the skin was dissected on centerline, brain injector (Harvard Apparatus) was inserted with a depth of 2.5 mm into a position of 0.2 mm and 1.2 mm in the back and lateral direction of bregma, respectively. The brain injector was immobilized by dental cements. Three day later, mouse was anesthetized with face mask using 2% isoflurane (Tocoris) and gas mixture (70%/30%) of nitrogen and oxygen, and body temperature was maintained at 37 ± 0.5°C using heating pad and lamp. Mice were randomly divided into two groups (control and test), and each group consists of five mice. Using brain injector, Five µl of PBS and 5 µl of hSTC2 (100 ng/5 µl) were intracerebroventricularly (I.C.V) injected to control and test group, respectively. Midline cervical cleft was incised to expose external carotid artery, and

5.0 nylon suture (Ethicon, Edinburg, UK) in a length of 9.0 mm, of which the tip was blunt with heat treatment was inserted into internal carotid artery through external carotid artery, blocking blood flow to middle cerebral artery. After 60 min, blood flow was recovered by removal of nylon. After focal cerebral ischemia for 24 hrs, mice were sacrificed and their brains were extracted. For coronal section, the brain tissues were cut from frontal lobe in a thickness of 1 mm using a brain matrice (Harvard Apparatus). Each fragment was incubated in 2% TTC (2,3,5-triphenyltetrazolium chloride) at 37°C for 15 min, and stained. Through scanning with a scanner (1,200 dpi; Hewlett-Packard), the images were analyzed using ImagePro-Plus software (Media Cybernetics).

### Results

### Effect of Stanniocalcin 2 (STC2) on Kainic acid(KA)-Inducible Neuronal Death

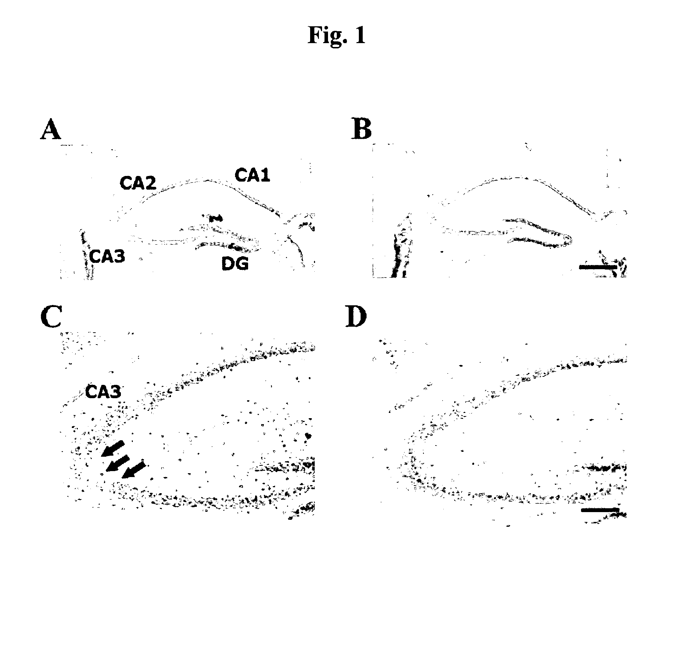

In this study, the present inventors examined whether pyramidal neuronal death (practically, apoptosis of neuronal cells) in hippocampal CA3 region by KA is inhibited by STC2. Five µl of mixture solution (containing 0.1 µg kainic acid and 100 ng hSTC2 in 5 µl solution) was intracerebroventricularly (I.C.V) injected to male ICR mouse with weight of 23-25 g. After injection for 24 hrs, the brain tissues were extracted. The brain tissue sections were stained with cresyl violet to observe neuronal death in hippocampal CA3 region. As a result, it was demonstrated that pyramidal neuronal death in hippocampal CA3 region was produced in one group treated with KA alone, whereas inhibited in the other group treated with both KA and STC2 (Fig. 1). According to the present invention, it could be appreciated that STC2 plays a protective role in excessive neurotoxicity.

### Effects of Stanniocalcin 2 (STC2) on Neurogenesis

It has been known as neurogenesis that neuron in a part of brain is proliferated and differentiated although differentiation of neuronal cell is finished. Neurogenesis is generated in subgranular zone (SGZ) beneath granular cell layer (GCL) of dentate gyrus (DG) in hippocampus which is responsible for memory and cognitive function in brain, and is known to be promoted by learning.

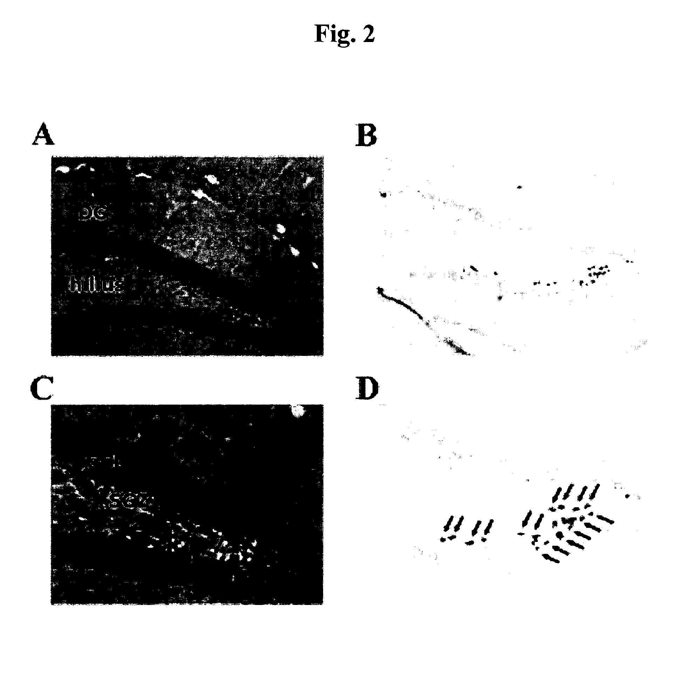

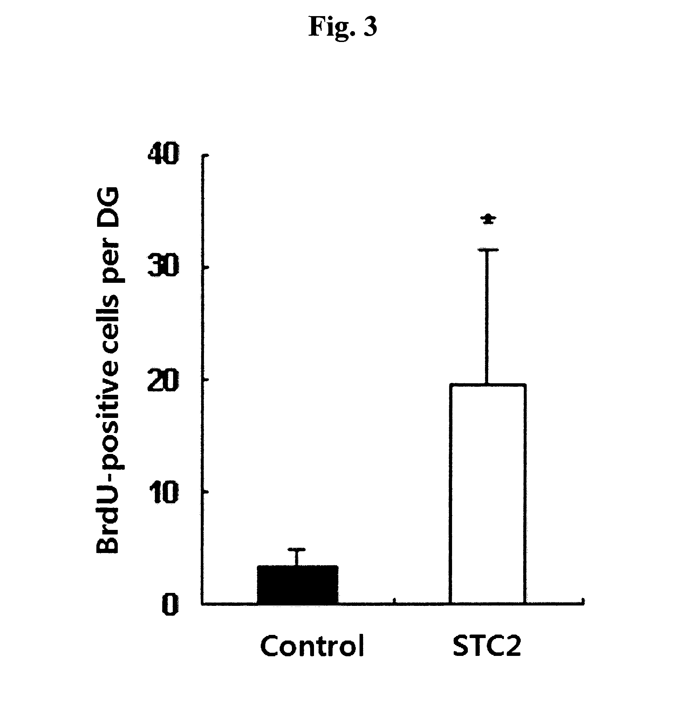

STC2 (10 nM) was intracerebroventricularly (I.C.V) injected to male ICR mouse with weight of 23-25 g, and bromodeoxyuridine (BrdU; 100 mg/kg) was intraperitoneally injected to male ICR mouse with weight of 23-25 g. After injection for 24 hrs, the brain tissues were extracted and subjected to BrdU immunohistochemistry. As a result, it could be demonstrated that BrdU-immunopositive cells in a group treated with STC2 are increased in SGZ of hippocampus compared to control (Fig. 2 and Fig. 3). According to the present invention, it could be appreciated that STC2 promotes neurogenesis.

### Effects of Stanniocalcin 2 on Y-maze Test in Mouse Treated with Kainic Acid

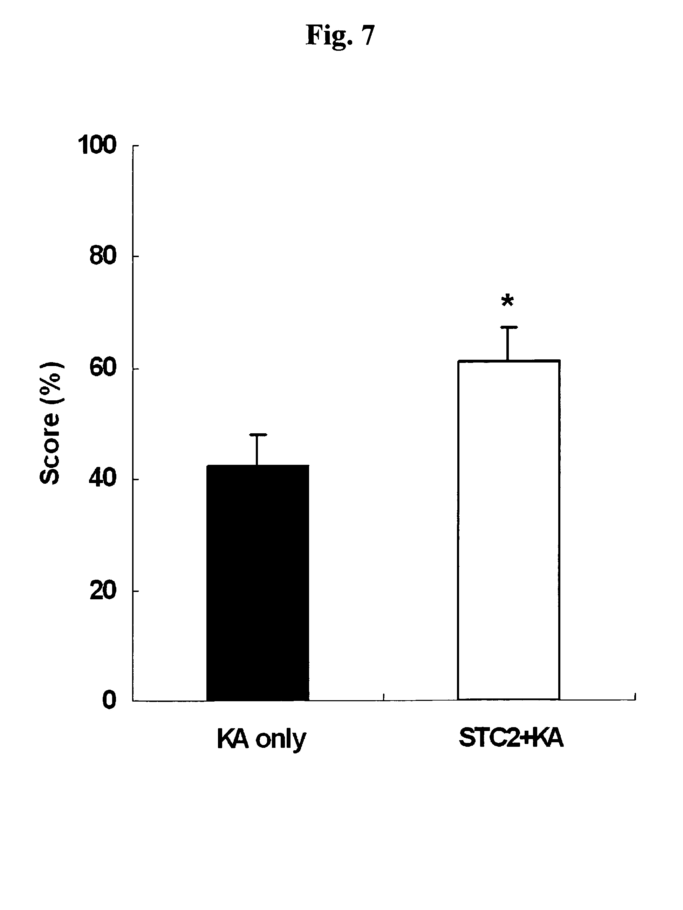

The present experiment is carried out to examine place memory function using research and curiosity which are basic characteristics in rodents. Memory score was 42.5 ± 5.4% in KA (kainic acid) alone-treated group and 61.3 ± 6.3% in the group treated with both hSTC2 and KA, suggesting that memory score decreased by KA is remarkably enhanced by hSTC2 (Fig. 7).

### Effects of Stanniocalcin 2 on Water Finding Test in Mouse Treated with Kainic Acid

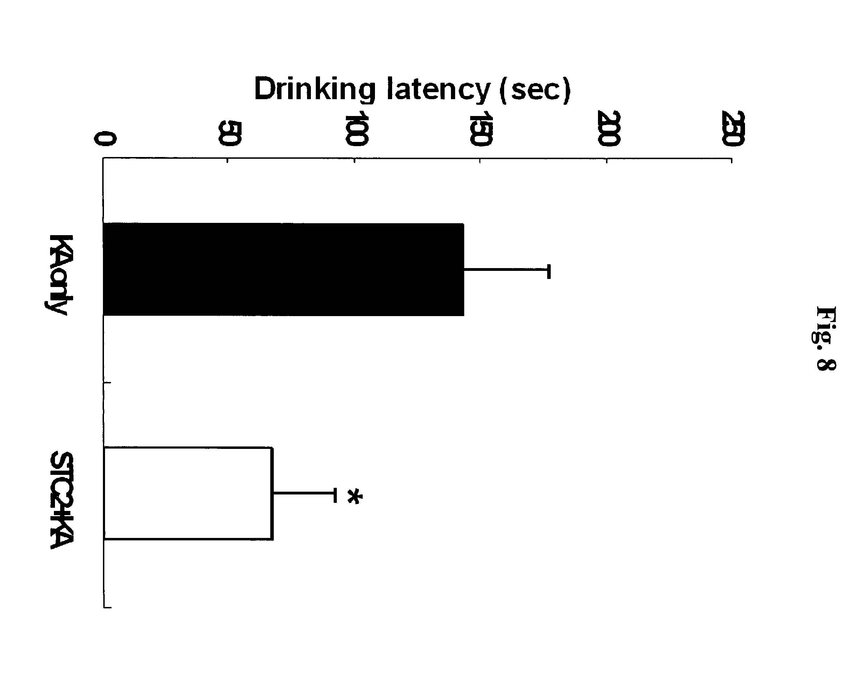

The present experiment is carried out to estimate learning, place memory and working memory. In high level of learning and memory function, drinking latency is relatively short. Drinking latency in hSTC2-treated group (67 ± 25 sec) is significantly decreased compared to that in KA alone-treated group (143 ± 34 sec) (p < 0.05) (Fig. 8).

### Effects of Stanniocalcin 2 on Forced Swim Test in Mouse Treated with Kainic Acid

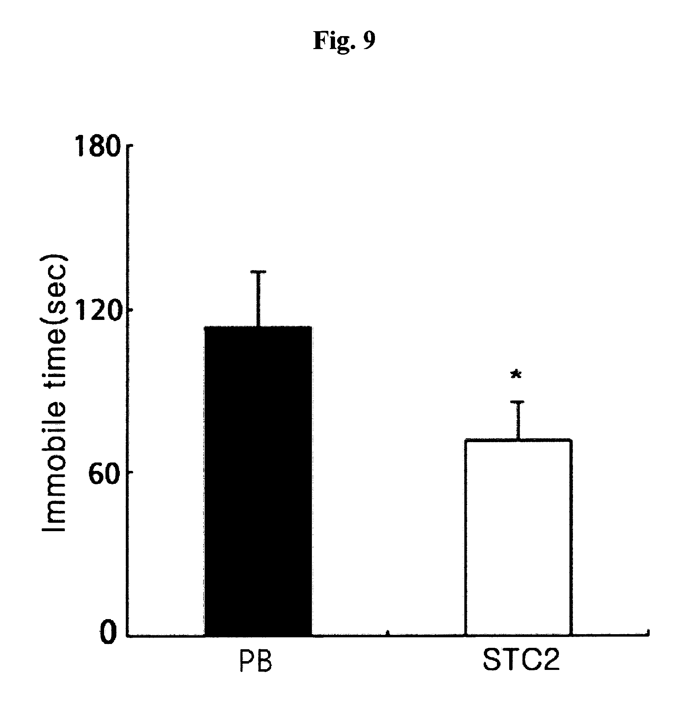

The present test is commonly utilized as a depression animal model for observation and assessment of depression-related behavior. The longer immobile time criterion, the higher helplessness estimated from the test. Immobile time in hSTC2-treated group (71 ± 15 sec) is significantly reduced compared to that in a KA alone-treated group (113 ± 21 sec) (p < 0.05) (Fig. 9).

### Effects of Stanniocalcin 2 on Brain Impair Caused by Transient Focal Cerebral Ischemia and Middle Cerebral Artery Occlusion (MCAO)

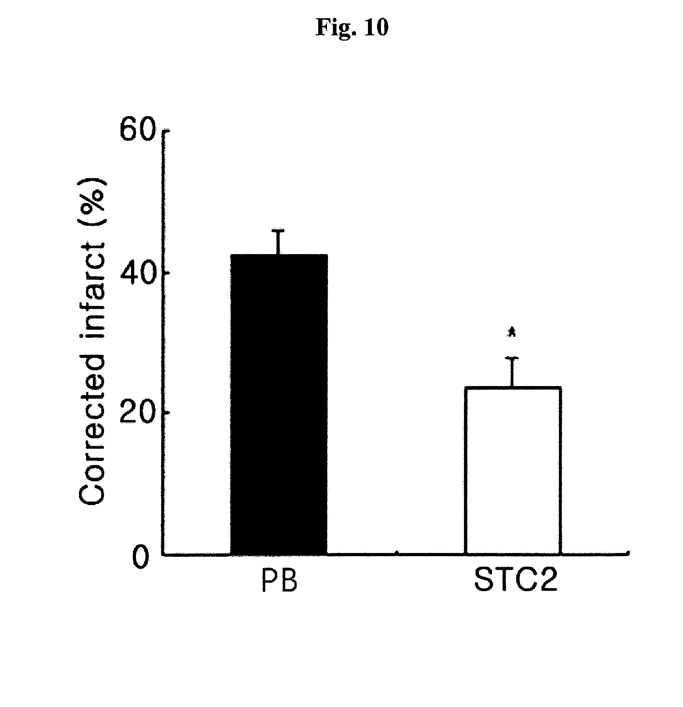

The present experiment is carried out to determine whether injection of hSTC2 decreases cerebral infraction and neurological deficit. The size of cerebral infraction in hSTC2-treated group (23.8 ± 4.2 sec) is significantly reduced compared to that in KA alone-treated group (42.2 ± 3.4 sec) (p < 0.05) (Fig. 10).

Having described a preferred embodiment of the present invention, it is to be understood that variants and modifications thereof falling within the spirit of the invention may become apparent to those skilled in this art, and the scope of this invention is to be determined by appended claims and their equivalents.

## Claims

1. A composition for preventing or treating a brain disease, which comprises stanniocalcin 2 as an active ingredient.

2. A composition for improving a cognitive function, which comprises stanniocalcin 2 as an active ingredient.

3. The composition according to claim 1 or 2, wherein the composition is a pharmaceutical composition or a food composition.

4. The composition according to claim 1 or 2, wherein the composition has a protective activity for neuronal cells.

5. The composition according to claim 4, wherein the protective activity for neuronal cells is exhibited by inhibiting a neuronal apoptosis.

6. The composition according to claim 1 or 2, wherein the composition has an activity for promoting a neurogenesis.

7. The composition according to claim 1, wherein the brain disease is selected from the group consisting of a neurodegenerative disease, an ischemia-reperfusion injury and a mental disorder.

8. The composition according to claim 7, wherein the mental disorder is depression or manic depression.

9. The composition according to claim 2, wherein the cognitive function is learning ability, memory or concentration.

10. A method for preventing or treating a brain disease, which comprises administering to a subject a pharmaceutical composition comprising stanniocalcin 2 as an active ingredient.

11. A method for improving a cognitive function, which comprises administering to a subject a pharmaceutical composition comprising stanniocalcin 2 as an active ingredient.

12. The method according to claim 10 or 11, wherein the composition is a pharmaceutical composition or a food composition.

13. The method according to claim 10 or 11, wherein the composition has a protective activity for neuronal cells.

14. The method according to claim 13, wherein the protective activity for neuronal cells is exhibited by inhibiting a neuronal apoptosis.

15. The method according to claim 10 or 11, wherein the composition has an activity for promoting a neurogenesis.

16. The method according to claim 10, wherein the brain disease is selected from the group consisting of a neurodegenerative disease, an ischemia-reperfusion injury or a mental disorder.

17. The method according to claim 16, wherein the mental disorder is depression or manic depression.

18. The method according to claim 11, wherein the cognitive function is learning ability, memory or concentration.
